# EUROPEAN PATENT APPLICATION

(11) **EP 1 162 184 A1**
(43) Date of publication of application: **12.12.2001**
(21) Application number: 00810499.4
(22) Date of filing: 08.06.2000
(51) Int. Cl.: C07C 2/86, B01J 19/08

(54) **Gasoline production by dielectric barrier discharge**

(71) Applicant: ABB RESEARCH LTD., 8050 Zürich (CH)
(72) Inventor: Eliasson, Baldur, Dr., 5413 Birmenstorf (CH); Zhang, Kui, Tianjin 30072 (CN); Kogelschatz, Ulrich, 5212 Hausen (CH)
(74) Representative: ABB Patent Attorneys

(57) **Abstract**

A method of transforming a normally gaseous composition containing a mixture of normally gaseous hydrocarbons and carbon dioxide into a product stream containing at least one normally liquid hydrocarbon, wherein the mixture of normally gaseous hydrocarbons contains at least saturated and/or unsaturated C4-hydrocarbons; the method comprising the steps of feeding the composition into a reactor including a first electrode means, a second electrode means and at least one layer of a normally solid dielectric material positioned between the first and the second electrode means; submitting the composition within the reactor in the presence of a normally solid catalyst to a dielectric barrier discharge, wherein the normally solid catalyst is a member selected from the group of zeolites, modified zeolites and zeolite-like materials; and controlling the dielectric barrier discharge to convert the normally gaseous composition into the product stream.

## Description

### Background of the Invention

The present invention relates to a method of transforming a normally gaseous composition containing a mixture of normally gaseous hydrocarbons and carbon dioxide into a product stream containing at least one normally liquid hydrocarbon.

A major objective in today society is the economic utilization of fossil energies and natural resources such as petroleum, and therefore, the improvement of energy efficiency and waste reduction in processes related to oil refinery and petrochemical industry. Furthermore, the man-made emissions of greenhouse gases such as carbon dioxide, which are suspected of being at least partially responsible for the global warming of the atmosphere, represents another major problem facing mankind. One possible approach to either reduce the amount of waste material or low grade material derived from oil refinery and petrochemical processes as well as to mitigate the emissions of carbon dioxide would be to recycle them in a chemical process to form useful and high quality products.

### Prior Art

Petroleum refining is a major industry in modern society which processes world-wide some 80 million barrels of crude oil per day. As a basic principle, crude oil is first separated into various fractions by a primary distillation. Gas, which is the lightest fraction, is produced as the top product from the atmospheric fractionation column; it consists of normally gaseous hydrocarbons with one to four carbon atoms, and in particular of butanes and butenes. Other fractions of the primary distillation that contain the main refinery products are, in order of ascending atmospheric boiling points, naphtha, kerosene, gas oil and residue.

In practice, the yield and quality of each fraction does not match market demand. Consequently, the less valuable heavy fractions must be converted into lighter ones, and the lighter fractions must be chemically modified to improve their properties. Examples of these conversion processes are catalytic reforming, catalytic cracking, thermal cracking, hydrocracking and alkylation. The products of these petroleum refining processes again include normally gaseous hydrocarbons containing butanes and/or butenes as by-products, which are in general separated from the desired components by way of fractionation.

Thus, gas streams consisting of normally gaseous hydrocarbons containing C4-hydrocarbons are produced from direct fractionation of crude oil and from fractionation of the products of various refinery conversion processes. Even if these gas streams could be used as fuel gas for refinery furnaces or could be compressed and sold as bottled gases for light heating applications in home heating and campers or for barbecues, most of them are, even today, dictated to be flared due to the high cost of processing, compressing or liquefying and shipping these gases.

The formation of normally gaseous hydrocarbons including C3- and, in particular, C4-hydrocarbons as undesired by-products are, furthermore, known in several petrochemical processes such as in Mobile's methanol to gasoline (MTG) and methanol to olefin (MTO) processes. As in the case of petroleum refining processes these normally gaseous mixtures are generally flared, and thus, valuable resources being wasted and carbon dioxide being emitted.

Several methods are described to convert normally gaseous hydrocarbons into higher boiling, preferably normally liquid, hydrocarbons. Most of these methods use thermal and/or catalytic activation for the transformation. One of the latter is described in US-4'414'423. However, there is still a need for economic methods of transforming mixtures of gaseous hydrocarbons particularly containing C4-hydrocarbons into higher normally liquid hydrocarbons.

Non-equilibrium plasma chemical processes occurring in the volume part of electrical non-equilibrium discharges have attracted a great deal of interest. Particularly, silent gas discharges have demonstrated their suitability for large-scale industrial applications, such as the generation of ozone. A characteristic of the silent discharge is the presence of a dielectric. Therefore silent gas discharges are also referred to as dielectric barrier discharges.

Recently, the utilization of greenhouse gases for the synthesis of methanol or methane in such silent gas discharge reactors has also been described. Thus, DE 42 20 865 describes a method and an apparatus for the hydrogenation of carbon dioxide leading in particular to methane or methanol by exposing a mixture of carbon dioxide and a substance containing hydrogen atoms, preferably hydrogen or water, to a dielectric barrier discharge. In the pending European Patent application No. 99810261 filed by the present assignee the synthesis of liquid hydrocarbons is disclosed, wherein a mixture of carbon dioxide and methane is exposed to a dielectric barrier discharge in the presence of a normally solid catalyst.

### Objects of the Invention

Accordingly, it is an object of the present invention to provide for a method of transforming a normally gaseous composition comprising a mixture of normally gaseous hydrocarbons, which contains particularly saturated and/or unsaturated C4-hydrocarbons, and carbon dioxide into a product stream containing at least one normally liquid hydrocarbon, which method minimizes or even avoids the disadvantages of the prior art.

Moreover, it is an object of the present invention to provide for a method of transforming a mixture of normally gaseous hydrocarbons including butanes and/or butenes into a product stream containing normally liquid hydrocarbons, which method can be carried out at low pressures, and preferably at low temperatures.

It is another object of the present invention to provide for a method of producing gasoline of high quality and having a high octane number starting from carbon dioxide and a mixture of gaseous hydrocarbons containing butanes and butenes.

It is a further object of the present invention to provide for a method of transforming a mixture of normally gaseous hydrocarbons containing butanes and/or butenes into a product stream containing normally liquid hydrocarbons, which method allows the recovery of valuable materials otherwise being flared.

It is still a further object of the present invention to provide for a method of transforming a normally gaseous composition comprising a mixture of normally gaseous hydrocarbons, which contains particularly saturated and/or unsaturated C4-hydrocarbons, and carbon dioxide into a product stream containing at least one normally liquid hydrocarbon, which method allows to avoids or at least minimizes the formation of hydrocarbons containing more than 11 carbon atoms as well as of polymer materials.

Further objects and advantages of the present invention will become apparent as this specification proceeds.

### Brief Summary of the Invention

We have found that the objects can be achieved according to a first general embodiment of the invention by a method as set forth in claim 1. Accordingly, the invention provides for a method of transforming a normally gaseous composition containing a mixture of normally gaseous hydrocarbons and carbon dioxide into a product stream containing at least one normally liquid hydrocarbon, wherein said mixture of normally gaseous hydrocarbons contains at least saturated and/or unsaturated C4-hydrocarbons; said method comprising the steps of feeding said composition into a reactor including a first electrode means, a second electrode means and at least one layer of a normally solid dielectric material positioned between said first and said second electrode means; submitting said composition within said reactor in the presence of a normally solid catalyst to a dielectric barrier discharge, wherein said normally solid catalyst is a member selected from the group of zeolites, modified zeolites and zeolite-like materials; and controlling said dielectric barrier discharge to convert said normally gaseous composition into said product stream.

### Definitions, Detailed Description of Preferred Embodiments and Elements of the Invention

The term "about" as used herein before any numeral implies a variation of typically ± 10%.

The term "normal" with regard to boiling points, boiling ranges and the like indicates that these values are understood as being corrected for "normal conditions", i.e. a temperature of 25°C and an atmospheric pressure of 1013 mbar. In analogy, the term "normal" with respect to physical states of matter and the like indicates that they are referred to said "normal conditions".

The term "layer" is used herein to refer to any planar or curved stratum having a width dimension that is substantially larger than its thickness dimension; typically, the width:thickness ratio is at least 10:1 and generally well above that value.

In the context of the present invention the term "hydrocarbons" stands for substances consisting of hydrogen and carbon atoms, such as aliphatic hydrocarbons being either saturated, such as alkanes, or unsaturated, such as alkenes and alkynes, cycloaliphatic hydrocarbons being either saturated or unsaturated, and/or aromatic hydrocarbons. The abbreviations such as "C4-hydrocarbons" or "C11-hydrocarbons" as used herein stands for hydrocarbons having the corresponding number of carbon atoms within their skeleton, i.e. four and eleven respectively. It is to be noted that the invention relates to any of the possible isomers and mixtures thereof. In analogy, the term "butanes and/or butenes" as used herein either imply the presence of just a single isomer being either a saturated or an unsaturated one as well as mixtures of some or all possible isomers of C4-hydrocarbons.

Sources of normally gaseous compositions containing a mixture of normally gaseous hydrocarbons including C4-hydrocarbons are, in particular, gas streams derived from direct fractionation of crude oil or the by-products gases from various refinery conversion processes such as fluidized catalytic cracking or steam cracking of wax distillates. Furthermore, mixtures of normally gaseous hydrocarbons including, in particular, C4-hydrocarbons, which are used in the present invention, stems from petrochemical processes such as Mobile's methanol to gasoline (MTG) and methanol to olefin (MTO) processes. Moreover, any waste and exhaust gases deriving from industrial processes such as the by-product gases from the Fischer-Tropsch synthesis, which gases contain a mixture of normally gaseous hydrocarbons including at least butanes and/or butenes, can be used for the present invention. If the aforementioned gaseous mixtures used for the present invention do not already contain carbon dioxide, the latter is either co-fed into the reactor for the inventive method or it is mixed with the mixture of normally gaseous hydrocarbons containing C4-hydrocarbons prior to the feeding of the inventive normally gaseous composition into the dielectric barrier discharge reactor. It is obvious, however, that purification steps prior to the feeding into the reactor might be necessary to remove other species present in trace amounts in the aforementioned normally gaseous compositions used according to the invention.

According to a preferred embodiment of the present invention the saturated and/or unsaturated C4-hydrocarbons are contained in the mixture of normally gaseous hydrocarbons in a molar concentration of at least about 5%, preferably of at least about 10% and most preferably of at least about 20%.

In a further preferred embodiment of the present invention the normally gaseous hydrocarbons and the carbon dioxide are contained in the normally gaseous composition at a molar ratio of carbon dioxide:mixture of normally gaseous hydrocarbons of between about 1:1 to about 1:4, preferably between about 1:2 to about 1:3.

The normally solid catalyst is a "shape-selective catalyst" being selected from the group of zeolites, modified zeolites and zeolite-like materials and thus having an at least partially crystalline, typically completely crystalline, solid structure. The term "shape-selective catalyst" is intended to refer to a catalyst that owns a special structure containing channels and pores, respectively, to limit the diffusion of the reacting molecules and the formed product molecules through its framework. Only molecules with diameters smaller than the openings and apertures, respectively, of the channels can pass through the shape-selective catalyst. Moreover, an additional constraint is imposed by the size and shape of the apertures and channels with respect to possible transition states of the reaction.

Zeolites are crystalline aluminosilicates of group IA and group IIA elements such as sodium, potassium, magnesium and calcium with the empirical formula (I)

M_{2/n}O · (1-y)Al₂O₃ · ySiO₂ · wH₂O (I)

wherein
n is the valence of the cation M,
y the molar fraction of SiO₂ in the zeolite framework, and
w number of water molecules present in the
zeolite.
Zeolite structures are based on a three-dimensional framework of AlO₄ and SiO₄ tetrahedra (Si and Al defined as T atoms) which are interlinked through common oxygen atoms forming O-T-O-T-O bonds to give said three dimensional network through which channels run. Each AlO₄ tetrahedron in the framework bears a net negative charge which is compensated by a cation of the aforementioned group IA and group IIA elements. This cation is mobile and can be exchanged by other cations. In the interior of the channels are water molecules and the mentioned mobile cations. Criteria to distinguish zeolites and zeolites structures, respectively, are, inter alia, the number of either T- or O-atoms forming the rings and openings, respectively, controlling diffusion through the channels as well as the molar ratio of SiO₂/Al₂O₃, in short the Si/Al ratio.

"Modified zeolites" as used herein refers to natural or synthetic zeolites which have been modified by either cation or ion exchange, thermal treatment, hydrothermal treatment, treatment with inorganic or organic acids, treatment with inorganic or organic F materials, i.e. inorganic or organic compounds containing fluor or fluoride, as well as with organic silica materials, and, in particular, by an isomorphic substitution of the T atoms. Such an isomorphic substitution of the T atoms, e.g. leading to silicoalumino-phosphates, can be effected particularly by changing the parameters of the zeolite syntheses or by "post synthesis" modifications. Thus, various T atoms can be introduced into a zeolite framework during the synthesis step, such as Ga, Ge, Li, Be, B, Mg, Ti, Mn, Fe, Co, In, As and H. Such an introduction of a certain element and its concentration in a zeolitic framework usually occurs by mixing the different components in the synthesis gel. However, any other procedure for the introduction of the mentioned and other elements leading to modified zeolites are within the scope of the present invention. Moreover, the preparation method by simply mixing the different components as well as other procedures for the synthesis of modified zeolites are known by the person skilled in the art and need no further explanation herein. In analogy, "post synthesis" modifications of zeolites, i.e. modifications of existing natural or synthetic zeolites to effect a T-site substitution are also within the knowledge of the artisan and do not need to be further explained herein. Thus, various gaseous and liquid reactants as well as different techniques, such as hydrothermal techniques, have been developed for such a post synthesis T-site substitution. In particular, numerous reactants and techniques have been described for aluminium removal with simultaneous substitution by other elements such as Si, Ti, V, Fe, Sn, Ge and P.

"Zeolite-like materials" as used herein refers to normally solid materials, such as aluminophosphates, silicoaluminophosphates, metalloaluminophosphates and metal oxides containing OH groups, which have solid structures similar to zeolites and, therefore, which contain channels with sizes and shapes similar to channel sizes and shapes in zeolite structures. In particular, "zeolite-like materials" refer to crystalline solids which have zeolite-like structures as indicated by their incorporation in the "Atlas of Zeolite Structure Types" reported by Meier and Olson in "Atlas of Zeolites Structure Types", Zeolites 17(1996), 1-230 (report being incorporated herein for all purposes by way of reference). Examples of those isotypic and homeotypic species having zeolite-like structures are reported, in particular, in Table 3 on page 14 of the aforementioned reference.

In another preferred embodiment of the present invention the normally solid catalyst has an at least partially crystalline solid structure with at least one 10-ring channel or with at least one 12-ring channel within its structure. The use of such a zeolite, modified zeolite or zeolite-like material as catalyst in the inventive method, limits the growth of the hydrocarbon chain and thus inhibits the undesired formation of solid polymer. Consequently, an increased production and yield, respectively, of liquid fuel, in particular of normally liquid hydrocarbons, results. Moreover, the application of shape-selective catalysts for the inventive method leads to a large amount of branched hydrocarbons, in particular branched normally liquid hydrocarbons, representing a higher-quality fuel due to a higher octane number.

Typical and preferred materials having a solid structure in accordance with the present invention, and thus having at least one 10-membered ring channel system or at least one 12-membered ring channel system within the structure, are given in the following by way of their structure type codes consisting of three capital letters. These structure type codes have been set up by the IUPAC Commission on Zeolite Nomenclature and are, inter alia, listed together with their basic structural features and specific examples of zeolites, modified zeolites and zeolite-like materials in the above-mentioned "Atlas of Zeolite Structure Types" reported by Meier and Olson in Zeolites 17(1996), 1-230. Thus, typical normally solid catalysts in accordance with the present invention are of the structure type AEL, AFI, AFO, AFR, AHT, BEA, FER, LAU, LTL, MEI, MEL, MFI, MFS, MOR, MTT, STI, ZSM-12 and ZSM-18.

In a further preferred embodiment of the present invention, the at least one 10-ring channel has a diameter of between about 4 Å and about 7 Å, or the at least one 12-ring channel has a diameter of between about 4 Å and about 8 Å. Such a well-defined pore system further increases the shape-selectivity of the normally solid catalyst since the pores and channels, respectively, must be capable to accommodate the reactants, i.e. the normally gaseous composition, and in particular carbon dioxide and the normally gaseous hydrocarbons or fragments thereof. Moreover, an additional constraint is imposed by the pores and apertures of the channels which control the size and shape of the product molecules. Therefore, the preferred diameter of the at least one 10-ring channel between about 4 Å and about 7 Å or of the at least one 12-ring channel between about 4 Å and about 8 Å in accordance with the invention, leads to a precisely defined arrangement of AlO₄ and SiO₄ tetrahedra and thus to a shape-selective dielectric barrier discharge reaction with good reproducibility.

In a further preferred embodiment of the present invention the solid structure of the normally solid catalyst have at least two channel systems, wherein at least one channel system is a 10-ring channel or a 12-ring channel. Typically these at least two channel systems are interconnected, wherein, however, the channel intersections are free of super cages and β-cages, respectively, as well as free of α-cages. These super cages and α-cages interconnecting the different channels have diameters which are larger than those of the channels and the apertures of the channels, respectively. Therefore, the preferred embodiment of the invention, in which the solid structure of the normally solid catalyst is free of super cages further increases the shape-selectivity of the transformation by further defining the catalyst.

In accordance with the present invention, the normally solid catalyst has preferably a Si/Al ratio of higher than 6, more preferably higher than 15. As indicated, the Si/Al ratio is a criterion to distinguish different zeolites, modfified zeolites and zeolite-like materials. Moreover, the Si/Al ratio strongly influences important properties of the inventive solid catalyst. Thus, the preferred Si/Al ratio indicated above leads to higher thermal and higher hydrothermal stability of the inventive solid catalyst. This is of particular benefit with respect to the regeneration of the normally solid catalyst since water might be formed during the regeneration process of the catalyst and as a by-product in the inventive dielectric barrier discharge reaction as well as small amounts of carbon deposits and/or polymeric material and hydrocarbons having a carbon chain built by 11 or more carbon atoms, respectively.

The increased hydrothermal stability of the aforementioned preferred embodiments of the present invention thus minimizes and/or avoids damages to the solid catalyst under the reaction conditions caused by the eventual formation of water. Moreover, the preferred Si/Al ratio particularly contributes to the increase of the structure destructive temperature of the inventive normally solid catalysts allowing a standard regeneration taking place at about 600°C. Thus, high silica Zeolite catalysts such as HZSM-5 are thermally stable up to 1000°C.

Preferred normally solid catalysts used in the present invention are a member of the group selected from zeolites and modified zeolites having a crystalline solid structure selected from BEA, FER, MEL, MFI, MOR, MTT and ZSM-12 as specified and defined in the above-mentioned "Atlas of Zeolite Structure Types" reported by Meier and Olson in Zeolites 17(1996), 1-230. Typical examples of those preferred catalysts are the so-called pentasil-type zeolites, such as Ferrierite and ZSM series catalysts, e.g. ZSM-5, ZSM-23, ZSM-20 and ZSM-11, and the corresponding modified zeolites as defined above.

In particular the use of ZSM-5 zeolites, and here preferably the NaZSM-5 and more preferably the HZSM-5, is highly preferred as the normally solid catalyst for the present invention. The shape-selective Zeolite ZSM-5 is a synthetic high-silica zeolite with a high thermal stability. The MFI-type topology shows a three-dimensional 10-member ring channel system having no super cages in its structure.

The use of a ZSM series zeolite catalyst for the inventive method leads to a product stream containing normally liquid hydrocarbons, as well as syngas, normally gaseous hydrocarbons, and higher hydrocarbons, i.e. having a carbon chain of at least 11 carbon atoms (C>11), and/or polymeric material. The products formed in the reaction over ZSM series catalysts, in particular, contain a large amount of branched hydrocarbons having a carbon chain of 6 to 10 carbon atoms improving the octane value and, thus, the quality of gasoline and liquid fuel, respectively. Beneficial for the former is, moreover, the formation of some normally liquid aromatics and oxygenates in the inventive method. The formation of polymer materials and higher hydrocarbons (>C11) is strongly restricted over ZSM catalysts because of their special pore structures and catalytic properties.

The use of zeolite-like materials and, in particular, the use of zeolites and modified zeolites as the normally solid catalyst offers, moreover, the advantage of having high concentrations of OH groups on the surfaces, i.e. on the outer surfaces of the zeolite structure as well as within the zeolite cavities and channels, respectively. In addition to the high concentration of OH groups on zeolite surfaces, an important characteristic of zeolites is the natural coulombic field formed within the zeolite framework. Within this context, it should be noted that both the concentration of OH groups and the strength of the natural coulombic field are controllable and adjustable. Generally, these two features allow the zeolites to easily respond to an external electric field, i.e. the zeolite becomes electrically charged more easily. The control of the dielectric barrier discharge according to the invention allows though to control these charges and electrostatic fields and, therefore, to control zeolite activity and selectivity in the conversion of a normally gaseous composition into a product stream containing at least a normally liquid hydrocarbon.

As already indicated, the use of zeolite-like materials, and in particular zeolites and modified zeolites, as the normally solid catalyst inhibits the formation of carbon black, in particular, the precipitation of carbon black on the surface of the dielectric material, thus, allowing a long term operation of the dielectric-barrier discharge reactor. Moreover, the use of aforementioned catalysts for the inventive method limits the growth of the hydrocarbon chain. Consequently, an increased yield of normally liquid hydrocarbons having a normal boiling range of between about 50°C and about 210°C results and the formation of higher hydrocarbons (C>11) and polymeric material are suppressed. Furthermore, applying shape-selective catalysts leads to an increased tendency to form branched hydrocarbons, in particularly, of normally liquid branched hydrocarbons representing a high-quality fuel.

A further advantage of zeolites is the possibility of a controlled incorporation of acid centers in the intracrystalline surface, e.g. during synthesis and/or by subsequent ion exchange reaction. Thus, at temperatures above 300°C, pentasils have acidities comparable to those of mineral acids. Preferably, the normally solid catalyst is an acid type zeolite catalyst, such as HZSM-5 with varying Si/Al ratio.

In a further preferred embodiment of the invention, the normally solid catalyst comprises at least one substance selected from the group of metal ions and group IA, IIa, IB, IIb and VIII elements of the periodic table. The latter mentioned elements, i.e. alkali, earth alkali elements as well as the elements of the zinc and the copper group and the iron groups of the periodic table can be present either in ionic or atomic form. Those normally solid catalysts are synthesized by procedures generally known to the man skilled in the art, such as any type of ion exchange reactions or impregnations, in case followed by subsequent thermal activation or reduction. Examples of those solid catalysts are the Fe-ZSM-5, Fe/H-ZSM-5, Mo/H-ZSM-5, and H-GaAlMFI. The use of bifunctional catalysts, in particular the introduction of an hydrogenation function in an acidic zeolite, such as the incorporation of Pt²⁺ and Pd²⁺ ions may further prevent or lower the formation of carbon deposits.

In a further preferred embodiment of the invention, the method comprises the additional step of recirculating the normally gaseous components of the product stream into the reactor.

### Brief Description of the Drawing

For a better understanding of the nature and scope of the present invention - and not to limit the invention - preferred embodiments are described in more detail in the following by reference to the drawing, in which:

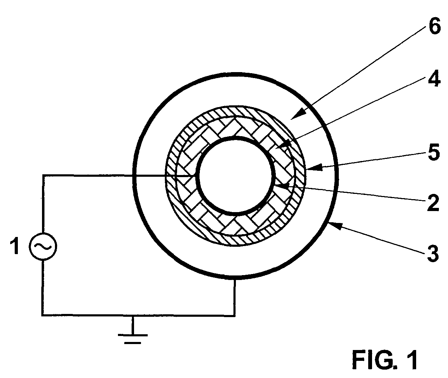

Fig. 1 is a diagrammatic cross sectional view of a preferred dielectric barrier discharge reactor configuration used for a preferred embodiment of the inventive method;

### Detailed Description of the Drawing

The dielectric barrier discharge is a high pressure non-equilibrium discharge which occurs when alternating voltages are applied to a gas space between two electrodes separated by a non-conducting medium. The amplitude of the alternating high voltage has to be high enough to cause electrical breakdown in the gas space. Fig. 1 shows schematically a cross sectional view of a dielectric barrier discharge reactor according to the invention. The high voltage AC generator 1 is connected to the first electrode 2 and to the second grounded electrode 3 both having an essentially cylindrical form. The electrodes are generally made of corrosion-resistant metals or alloys or of materials covered by at least one layer of an electrically conducting substance. Electrode 2 forms an inner shell and Electrode 3 forms an outer shell. The dielectric layer 4 is typically a glass, quartz or ceramic tube having a thickness of between about 0.1 mm and about 5 mm and covers the effective surface of electrode 2. The shape-selective catalyst 5 shown in Fig. 1, is also formed in essentially cylindrical form and is provided to cover the dielectric layer 4. Typically, the dielectric tube 4 serves as support for the solid catalyst 5. So, the solid catalyst 5, typically in powder form, is disposed in a piece of gas-permeable quartz fleece and wrapped around the outer surface of the dielectric tube 4, i.e. the surface of the dielectric tube 4 facing towards the electrode 3. Further catalyst support arrangements preferably used for the present dielectric barrier discharge reaction are described in EP-899'010 filed by the present assignee. It is obvious that the form and the size of the solid catalyst, i.e. whether it is applied in powder form or as grains of different sizes and the manner by which the catalyst is supported, i.e. by means of the dielectric material and by means of an additional support respectively, can be modified within the scope of the present invention.

The normally gaseous composition passes in the axial direction through the essentially annular discharge gap 6, where it is exposed to the dielectric barrier discharge. The dielectric barrier discharge is effected by an AC potential applied between the first electrode and the second electrode means. The preferred AC potential being in the range of from about 6 kV to about 100 kV and the frequency of the AC potential preferably being in the range of from about 50 Hz to about 1 MHz. Generally a specific electric power up to about 1-25 kW/m² of electrode area is fed into the discharge reactor by automatically adjusting the amplitude and frequency of the applied voltage. As indicated above, an operating pressure in the range of from about 0.01 bar to about 30 bar, preferably from about 0.1 bar to about 10 bar, at an operating temperature up to about 400°C, preferably up to about 200°C, is maintained in the reactor. The normally gaseous mixture is passed through said reactor preferably at a flow rate of from about 0.1 m³ per hour and per m² of electrode area to about 10 m³ per hour and per m² of electrode area.

When the amplitude of the applied AC electric field reaches a critical value, breakdown is initiated in the gas and a current flows from one electrode to the other. Once breakdown is initiated at any location within the discharge gap, charge accumulates on the dielectric leads to the formation of an opposite electric field. This opposite electric field reduces the external electric field within the gap and interrupts the current flow in a few nanoseconds to form short-lived microdischarges. The duration of the current pulse relates to pressure and properties of gases involved and the dielectrics applied. A large number of such microdischarges will be generated when a sufficiently high AC voltage is applied. The principal advantages of dielectric barrier discharge are that non-equilibrium plasma conditions are established at about atmospheric pressure and that the entire electrode area is effective for discharge reactions.

The inventive method leads to the formation of high quality gasoline, i.e. a mixture of normally liquid hydrocarbons having a high octane number due to large amounts of branched hydrocarbons, as well as to aromatics and oxygenates. Moreover, syngas, i.e. a normally gaseous mixture of carbon monoxide and hydrogen, is also produced as by-product in the inventive method.

Advantageously, the present invention allows the utilization and recovery of mixtures containing normally gaseous hydrocarbons including butanes and/or butenes otherwise often being flared as well as their transformation into high value products. Moreover, the present invention contributes to decrease of emissions of carbon dioxide in the atmosphere due to its incorporation in the inventive method. Thus, the inventive method leads to a better carbon resource utilization.

Although certain preferred embodiments of the invention have been described herein, it will be apparent to those skilled in the art to which the invention pertains that modifications and variations of the described embodiments may be made without departing from the spirit and scope of the invention.

### List of Reference Signs

- 1: high voltage AC generator
- 2: first electrode means
- 3: second electrode means
- 4: dielectric layer, dielectric material
- 5: catalyst
- 6: discharge gap

## Claims

1. A method of transforming a normally gaseous composition containing a mixture of normally gaseous hydrocarbons and carbon dioxide into a product stream containing at least one normally liquid hydrocarbon, wherein said mixture of normally gaseous hydrocarbons contains at least saturated and/or unsaturated C4-hydrocarbons; said method comprising the steps of:
feeding said composition into a reactor including a first electrode means, a second electrode means and at least one layer of a normally solid dielectric material positioned between said first and said second electrode means;
submitting said composition within said reactor in the presence of a normally solid catalyst to a dielectric barrier discharge, wherein said normally solid catalyst is a member selected from the group of zeolites, modified zeolites and zeolite-like materials; and
controlling said dielectric barrier discharge to convert said normally gaseous composition into said product stream.

2. The method of claim 1 wherein said saturated and/or unsaturated C4-hydrocarbons are contained in said mixture of normally gaseous hydrocarbons in a molar concentration of at least about 5%, preferably of at least about 10% and most preferably of at least about 20%.

3. The method of claim 1 or 2 wherein said normally gaseous hydrocarbons and said carbon dioxide are contained in said normally gaseous composition at a molar ratio of carbon dioxide:mixture of normally gaseous hydrocarbons of between about 1:1 to about 1:4, preferably between about 1:2 to about 1:3.

4. The method of any of claims 1 to 3 wherein said normally solid catalyst has an at least partially crystalline solid structure with at least one 10-ring channel or with at least one 12-ring channel.

5. The method of any of claim 4 wherein said at least one 10-ring channel has a diameter of between about 4 Å and about 7 Å, or wherein said at least one 12-ring channel has a diameter of between about 4 Å and about 8 Å.

6. The method of any of claims 1 to 5 wherein said solid structure of said normally solid catalyst is free of super cages.

7. The method of any of claims 1 to 6 wherein said normally solid catalyst has a Si/Al ratio of higher than 6, preferably higher than 15.

8. The method of any of claims 1 to 7 wherein said normally solid catalyst is a zeolite or a modified zeolite having a crystalline solid structure selected from BEA, FER, LTL, MEL, MFI, MOR, MTT and ZSM-12.

9. The method of any of claims 1 to 8 wherein said normally solid catalyst comprises at least one substance selected from the group of metal ions and group IA, IIa, IB, IIb and VIII elements of the periodic table.

10. The method of any of claims 1 to 9 comprising the additional step of recirculating normally gaseous components of said product stream into said reactor.
